# EUROPEAN PATENT APPLICATION

(11) **EP 0 585 983 A2**
(43) Date of publication of application: **09.03.1994**
(21) Application number: 93201932.6
(22) Date of filing: 01.07.1993
(51) Int. Cl.: C12N 15/85, C12N 15/43, A61K 48/00, C12N 15/12, C12N 9/16, C12N 9/50, C12N 15/67

(54) **Nucleotide vector comprising a translation inhibition factor gene, and IRES region and a desired gene**

(30) Priority: 03.07.1992 IE 102404
(71) Applicant: Q.B.I. ENTERPRISES LIMITED, Omer 84965 (IL)
(72) Inventor: Zurr, Daniel, Herzliya (IL)
(74) Representative: Perani, Aurelio

(57) **Abstract**

1. A method for the in vitro production of a gene product, comprises: (a) preparing one or more recombinant nucleotide vector(s) capable of eukaryotic cell transfection, collectively containing 1) a gene encoding an eukaryotic translation inhibition factor of an eukaryotic translation factor (ETF), 2) at least one internal ribosome entry site (IRES) region, and 3) at least one gene encoding a polypeptide which it is desired to produce; (b) transfecting a eukaryotic cell culture with the said vector; (c) keeping the transfected cell culture under conditions suitable to promote cell activity; and (d) recovering the desired gene product from the said culture.

## Description

### Field of the Invention

The present invention relates to the synthesis of gene product. More particularly, the invention relates to a novel method for producing gene products, such as polypeptides and proteins, in eukaryotic cells with high yield, to vectors therefor, to products obtained thereby and to their use.

### Background of the Invention

The production of various proteins and peptides with commercial value is the basis for the establishment and the rapid growth of the genetic engineering industry. This is facilitated by utilizing sophisticated recombinant DNA technologies rather than conventional synthetic routes used by the chemical industry, which are inapplicable. At the core of these technologies is the in vivo fermentation of microorganisms, typically a bacteria, containing the right gene (in an appropriate expression vector) encoding the target protein. The fermentation is followed by "harvesting" the genetically engineered microorganisms and isolating the desired product through a series of separation and purification processes.

This method has some drawbacks. (a) Eukaryotic proteins expressed in microorganisms are often not properly folded; in particular, glycosilation and the proper conformation of complex proteins are not performed correctly. (b) Proteins expressed in large amounts often precipitate into insoluble aggregates (inclusion bodies) from which they can be recovered only by solubilization in denaturing agents. (c) Proteins which are secreted into the periplasmic space of the producer cell when produced in small amounts, often cannot be secreted there when produced in large amounts.

Compared with the above-mentioned bacterial systems, mammalian expression techniques have certain advantages, particularly for the expression of higher eukaryotic proteins. The expressed proteins are usually properly modified, and they almost always accumulate in the correct cellular compartment. So far, however, the mammalian expression techniques are practical for small and medium-scale work, but they are difficult and very expensive to perform on an industrial scale.

In eukaryotic organisms the synthesis of proteins is mediated by the ribosome scanning model. The (40S) ribosomal sub-unit (carrying Met-tRNA and various initiator factors) binds partially at the 5' end of the mRNA and then migrate in a 5' - 3' direction, stopping at the first AUG codon in favorable context for initiating translation.

In eukaryotics, all cellular cytoplasmic mRNA carry a 7-methylguanylate cap attached to their 5' end. The binding of the ribosomal sub-unit near or at the 5' end of the mRNA is facilitated by an interaction between the methylated cap structure and the cap binding protein complex (including the eIF-4F).

### Summary of the Invention

It is therefore clear that it is highly desirable to be able to provide a method by means of which production of gene products in eukaryotic cells could be carried out on an industrial scale and in an economic manner.

It is an object of the present invention to provide such a method which overcomes the limitations so far inherent to gene products production in eukaryotic cells.

It is another object of the invention to provide methods and recombinant nucleotide vectors which can be employed for this purpose.

It is still another object of the invention to provide methods by means of which cell activity can be controlled, under production conditions.

It should be understood that whenever the terms "gene product" or "polypeptide" or "protein" are used, they are meant to be used interchangeably, *mutatis mutandis.*

As used herein, the following terms have the meanings indicated hereinafter:
BiP - The human immunoglobulin heavy chain-binding protein (also known as GRP 78).

ETF - Eukaryotic translation Factor, e.g., the eIF-4F complex.

Extracellular production - the production of one or more selected polypeptide outside a host cell by the mimicking of cell activity in an appropriate medium.

Gene Product - a translation product, comprising - but not limited to - polypeptides and proteins.

In vitro production - the production of one or more selected polypeptides in eukaryotic cell lines.

In vivo therapeutic method - The use of recombinant techniques in living organisms, be it ex vivo, by removal of patient cells and their introduction to the patient after engineering thereof, or by direct administration to a patient.

IRES - Internal ribosome entry site.

Recombinant Nucleotide Vector - A vector including a recombinant nucleotide sequence (DNA or RNA).

TIF - Eukaryotic translation inhibition factor.

Transfection - The introduction of a vector containing a recombinant nucleotide sequence (DNA or an RNA) encoding one or more genes to be expressed into a host cell.

UTR - an IRES containing the 5' untranslated region of picornavirus or of BiP.

Vectors - Any autonomously replicating or integrating agent, including but not limited to, plasmids, viruses, phages and the like.

The present invention is directed, inter alia, to methods and vectors by means of which the cap-dependent translation of cell proteins is inhibited while allowing the cap-independent translation of engineered gene products to proceed.

Generally speaking, the method for the in vitro production of a gene product, according to the invention, comprises the steps of:
a) preparing one or more recombinant nucleotide vector(s) capable of eukaryotic cell transfection, collectively containing 1) a gene encoding a TIF of an eukaryotic translation factor (ETF), 2) at least one internal ribosome entry site (IRES) region, and 3) at least one gene encoding a polypeptide which it is desired to produce;
b) transfecting a eukaryotic cell culture with the said vector;
c) keeping the transfected cell culture under conditions suitable to promote cell activity; and
d) recovering the desired gene product from the said culture.

According to one embodiment of the invention the ETF comprises, or essentially consists of, the eIF-4F complex.

According to another embodiment of the invention, the recombinant nucleotide vector can be a DNA or an RNA vector.

In one embodiment of the invention, the recombinant nucleotide vector comprises, in the same vector, 1) a sequence encoding a TIF preceded by a eukaryotic cap-dependent promoter; and 2) a sequence encoding the desired gene product preceded by an IRES.

In a different embodiment of the invention the recombinant nucleotide vector comprises, in the same vector, 1) a sequence encoding a TIF preceded by an IRES; and 2) a sequence encoding the desired gene product preceded by an IRES.

Preferably, but non-limitatively, the gene encoding the TIF is the cDNA of the poliovirus 2A protease and the IRES region is that of a picornavirus or of BiP (UTR). As stated, a preferred picornavirus is the polio virus or Encephalomyocarditis virus (EMCV).

In another aspect, the method for the in vitro production of a gene product according to the invention, comprises the steps of:
a) preparing a first recombinant nucleotide vector, comprising a sequence encoding a TIF, preceded by a eukaryotic cap-dependent promoter;
b) preparing a second recombinant nucleotide vector, comprising at least a sequence encoding the desired gene product, preceded by an IRES;
c) transfecting a eukaryotic cell culture with both the said first vector and the said second vector;
d) keeping the transfected cell culture under conditions suitable to promote cell activity; and
e) recovering the desired gene product from the said culture.

In still another aspect, the method for the in vitro production of a gene product according to the invention comprises the stops of:
a) preparing a first recombinant nucleotide vector, comprising a sequence encoding a TIF, preceded by an IRES;
b) preparing a second recombinant nucleotide vector, comprising at least a sequence encoding the desired gene product, preceded by an IRES;
c) transfecting a eukaryotic cell culture with both the said first vector and the said second vector;
d) keeping the transfected cell culture under conditions suitable to promote cell activity; and
e) recovering the desired gene product from the said culture.

As will be more fully explained hereinafter, a preferred embodiment of the invention further comprises providing for external activation of the TIF expression, e.g., by mediating the external activation of TIF expression by metallothionein activity.

As stated, the gene product can be any useful material, e.g., a polypeptide or a protein.

Thus, according to one embodiment, the method according to the invention comprises transforming a eukaryotic cell with a recombinant DNA vector containing a gene encoding a TIF of the eukaryotic translation initiation factor(s), e.g. of eIF-4F, and an internal ribosome entry site (IRES) capable of mediating translation initiation to an internal ribosome-binding mechanism, followed by a gene encoding the polypeptide which it is desired to produce.

Thus, according to the invention, all host cell mRNA translation in the cell is inhibited or substantially prevented by the inactivation of the ETF, e.g., the eIF-4F complex, necessary to initiate such translation, whereby expression of normal cell proteins is substantially prevented, and mRNA translation is essentially possible only via an IRES. Thus, any gene encoding the desired polypeptide, which follows the IRES, is substantially the only gene which can be expressed in the cell as long as the ETF is inactivated.

Of course, as will be further explained hereinafter, the TIF activity and the gene expression, preceded by an IRES, can be split between two or more different vectors which are all introduced into the cell. Such a separation of functions between separate vectors is substantially equivalent to their inclusion in a single vector and, as such, also forms a part of the present invention.

It should be understood that, according to the invention, while expression of the polypeptide of choice is obtained in all cases by preceding the gene which codes for it with an IRES, expression of the TIF can be obtained either through an internal mechanism, by providing an IRES for the TIF, or TIF expression can be via a cap-dependent translation initiation. In the first case, the `TIF will be expressed continuously and, unless additional intervention is effected, as explained below, cell death will occur relatively quickly. In the second case, shut off of cell activities will lead also to the stoppage of TIF translation, thus allowing a periodic recovery of normal cell activities which prolongs the life of the cell.

As will be appreciated, shut off of normal cell translation activities makes all cell energy available to translate selected protein(s). Therefore, in some instances, irreversible shut off, via IRES-mediated TIF translation, will lead to such a dramatically improved expression of the selected protein, that cell death within a short time, at such a production level, will in certain cases be acceptable.

According to one embodiment of the invention, external control of TIF translation can be obtained, e.g. by activation through the addition of metal ions to the culture medium, as will be more fully described hereinafter. Accordingly, shut off of external activation, together with the choice of cap dependent or independent TIF translation, render the invention highly versatile and flexible.

As will be apparent to the skilled person, a great variety of genes encoding TIFs and IRESes as well as of proteins to be expressed, can be provided without exceeding the scope of the invention, and all recombinant nucleotide vectors which contain, sequentially, a gene encoding a TIF, an IRES and a gene encoding a polypeptide to be produced, are encompassed by the present invention. The TIF can be preceded either by an IRES or by a cap-dependent promoter, depending on the desired result, as explained herein.

Representative but non-limitative examples of such genes and sequences, as further more fully detailed hereinafter, include the IRES region of a picornavirus or of BiP, and the cDNA of the poliovirus 2A protease. Of course, non-functional sequences can be present between the various genes, as they do not affect the invention, and the term "sequential" is to be read in respect of functional sequences only.

Picornaviruses are mammalian plus strand RNA viruses whose genomes serve as mRNA. A large segment of the 5' non-translated region (UTR), approximately 400 nucleotides in length, promotes "internal" entry of ribosomes independent of the non-capped 5' end of the mRNA. Thus the viral mRNA is translated by a mechanism in which the ribosome binds directly to an internal site of the mRNA.

Poliovirus is a + stranded picornavirus whose polyprotein, encoded by an open reading frame spanning most of the viral RNA, is processed by the virus encoded proteases (a TIF). Picornaviruses cause a dramatic shut-off of host cell mRNA translation (within several hours after infection).

The mechanism of shut-off is related to inactivation of the eukaryotic translation initiation factor eIF-4F (an ETF). Poliovirus induces the cleavage of the P220 component of eIF-4F a factor that assists the binding of capped mRNA to the 40S ribosome as part of the translation initiation process. The cleavage of the P220 component is caused by the poliovirus protease 2A (a TIF). The virus strategy thus appears to be to prevent the cap dependent translation initiation used by cellular mRNAs, while allowing the cap independent internal translation of polio viral RNA.

Poliovirus 2A is a protease the mature form of which is generated by the cleavage of the precursor polyprotein of poliovirus at its N terminus by 2A itself and at C terminus by another protease 3C [Fl. Toyoda et al., 1988, Cell 45, pp. 761-770].

It is known that a cellular mRNA, encoding the immunoglobulin heavy chain binding protein (BiP) can be translated in poliovirus-infected cells at a time when cap dependent translation of all host cell mRNA is inhibited. The 5' leader of BiP (UTR) can directly confer internal ribosome binding to an mRNA in mammalian cells.

As will be appreciated by the skilled person, and as explained above, the shut-off of cell protein expression and the enhanced production of a selected protein will eventually lead to cell death. Thus, according to one embodiment of the invention, a cell can be used for a single production cycle and whatever polypeptide is produced before cell death can be recovered therefrom. Of course, stable cell lines can be engineered to produce a selected protein.

According to another preferred embodiment of the invention, on the other hand, the cells are utilized in a plurality of in vitro synthesis cycles, by adjusting the amount of 2A protease produced via an appropriate inducible promoter.

After transfection and subsequent expression of the 2A protease in the cells, the eIF-4F complex will be inactivated. As a consequence, capped cellular mRNA will not be able to associate with the polysomes, while the translation initiation by the internal ribosome binding mechanism of the selected protein mRNA will continue, as it is independent of the eIF-4F complex.

If the 2A protease is translated via a cap-dependent mechanism, as soon as the amount of 2A protease increases in the cell, its production will be depleted due to a slowdown in its translation. The cells will then switch back, temporarily, to normal activity, allowing for the recovery of metabolic processes necessary to ensure cellular viability. The production of the 2A protease will then also be restored, starting the whole cycle again, and so on. This is a so-called "on-off translation" mechanism.

As will be further apparent to the skilled person, the invention can also be employed for gene therapy, in vivo, in a therapeutic method which requires increased protein production in protein-deficient cells. Of course, it is also possible to produce a material which will ultimately cause cell death. This is useful, for instance, in cancer therapy. The produced material may be, e.g., a toxin or 2A protease, or any other suitable material which will eventually cause cell death. Thus an appropriate vector can be constructed, which contains the desired gene encoding the required protein, preceded by an IRES, which can be used in an appropriate pharmaceutical preparation in the transfection of cells of a patient in need thereof. Of course, any such vector and pharmaceutical preparations also form a part of the present invention.

It is also known to persons skilled in the art to target a specific vector to be transfected solely or principally into a specific type of cells. This, of course, is useful for a variety of therapeutic treatments employing the methods and vectors of the invention.

Also encompassed by the present invention is the extracellular production of a polypeptide, by employing an appropriate mRNA construct in a medium which mimicks cell conditions necessary for the translation of an IRES-preceded gene.

Thus, in one aspect, the invention isdirected to a method for the extracellular production of a gene product, which method comprises the steps of:
a) preparing a medium containing a recombinant nucleotide vector comprising at least one sequence encoding the desired gene product, preceded by an IRES;
b) providing in the medium substrates, ribosomes and promotors required for effecting translation;
c) keeping the medium under conditions suitable to promote gene expression; and
d) recovering the desired gene product from the said medium.

### Brief Description of the Drawings

- Fig. 1 is the restriction site map of the pSP64 poly (A) vector;
- Fig. 2 shows the PM² plasmid, the BamHI cloning site being enlarged;
- Fig. 3 illustrates the engineering of fragments to be inserted into the cloning site of plasmid pSP64;
- Fig. 4 illustrates the construction of the UTR and the BiP coding sequence, and the insertion of the enhanced domain from hMT into the PM² vector;
- Fig. 5 represents the activity following addition of acetylthiocholine substrate by the Ellman test (Example 1);
- Fig. 6 shows luciferase activities from extracts of Chinese Hamster Ovary (CHO) cells (Example 2); and
- Fig. 7 schematically illustrates the constructs of Examples 3 and 4.

### Detailed Description of Preferred Embodiments

By way of illustration, and without limiting the invention in any way, specific embodiments of the invention will be described hereinafter, with reference to the drawings.

### Example 1

### Expression of AcChoEase

### A. Construction of Expression Vectors

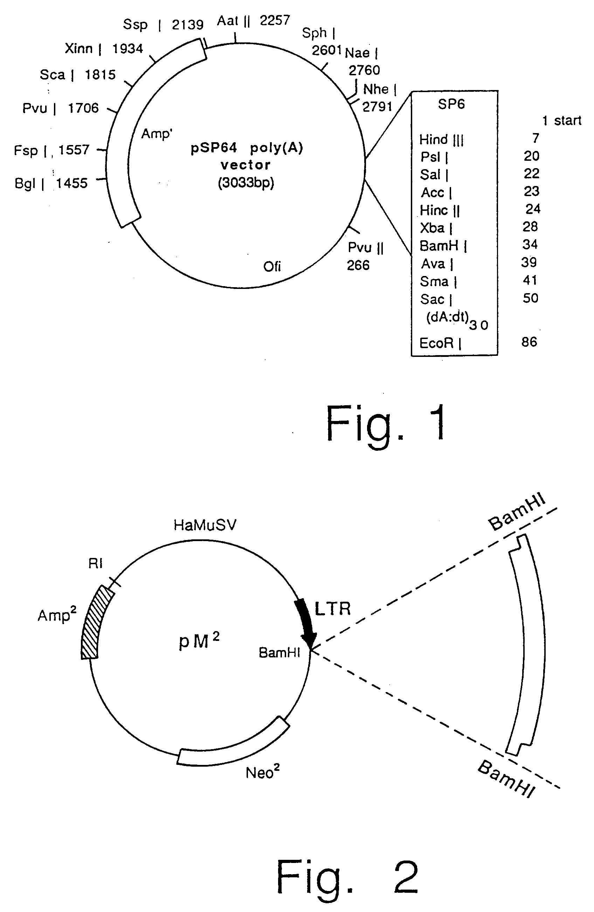

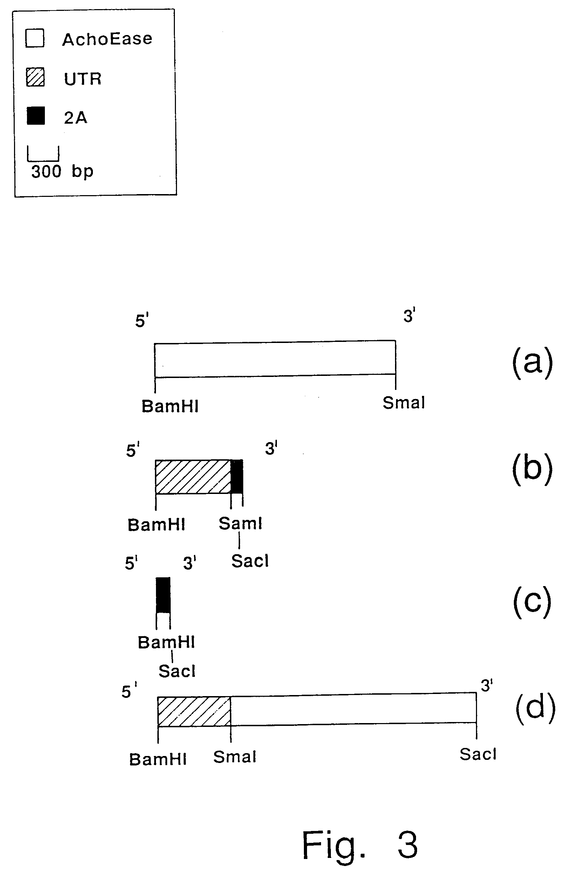

To construct an expression vector for shut-off of the host protein synthesis bearing the sequences of the UTR from BiP or poliovirus cDNA, and 2A domains from poliovirus cDNA, the pSP64 vector (Promega) was used, which contains a poly A sequence in its 3'-terminus (Fig. 1). Additionally, an efficient eukaryotic expression vector, PM², is used. The PM² plasmid (Fig. 2) contains the Harvey murine sarcoma virus long terminal repeat (LTR), the bacterial genes Neo and Amp which confer resistance to G418 and Ampicillin antibiotics respectively. PM² can therefore serve as an efficient selection element for creating stable transfected cell lines.

The following constructs were ligated into PM² at the BamHI cloning site (Fig. 3):

### Preparation of Construct 3(a)

For a control-capped reporter gene, the human Acetylcholinesterase (AcChoEase) gene was used. BamHI and SmaI sites were created at codons 1 and 2005 of AcChoEase cDNA, respectively. The fragment BamHI-SmaI was ligated to the pSF64 vector at the cloning site. The EcoRI site at the 3' end was modified into a BamHI site using the Klenow enzyme and a synthetic oligonucleotide linker including the restriction site specific for BamHI digestion. The composite BamHI-BamHI fragment containing the AcChoEase cDNA and poly A was inserted at the BamHI cloning site of PM².

### Preparation of Construct 3(b)

For shut-off of host protein synthesis the plasmid containing polio 5'UTR and the 2A protease coding region was constructed. The expression of 2A protease using this PM² plasmid was through cap-independent mechanism. BamHI and SmaI sites were created at codons 1 and 698 of poliovirus cDNA, respectively. Additionally, SmaI and SacI sites were created at codons 3386 and 3832, respectively. The two fragments thereof, BamHI-SmaI containing the UTR, and SmaI-SacI which contains the 2A domain, were ligated in frame into the cloning region of the pSP64 vector. The EcoRI site at the 3' end was modified into BamHI site as described above. The composite BamHI-BamHI fragment containing the UTR, 2A and Poly A was inserted at the BamHI cloning site of PM².

### Preparation of Construct 3(c)

2A protease coding region was constructed into PM². The expression of 2A protease using this plasmid was through cap-dependent mechanism. Expression of 2A protease through this mechanism results in shut-off of host protein synthesis, including the 2A protease. BamHI and SacI sites were created at codons 3386 and 3832, respectively. The BamHI-SacI fragment containing the coding sequence of 2A protease was ligated into the cloning site of the pSP64 vector. The EcoRI site at the 3' end was modified into the BamHI site as described above. The composite BamHI-BamHI fragment containing the 2A protease and poly A was inserted at the BamHI cloning site of PM².

### Preparation of Construct 3(d)

For an uncapped reporter, the UTR sequence was linked to the AcChoEase coding region. BamHI-SmaI sites were created at codons 1 and 698 of poliovirus cDNA, respectively. Additionally, SmaI and SacI were created at codons 1 and 2005 of AcChoEase cDNA. The two fragments BamHI-SmaI containing the UTR and SmaI-SmaI which contain the AcChoEase were ligated in frame into the cloning site of the pSP64 vector. The EcoRI site at the 3' end was modified into BamHI site as described above. The composite BamHI-BamHI fragment containing the UTR, AcChoEase and poly A was ligated at the BamHI cloning site of PM².

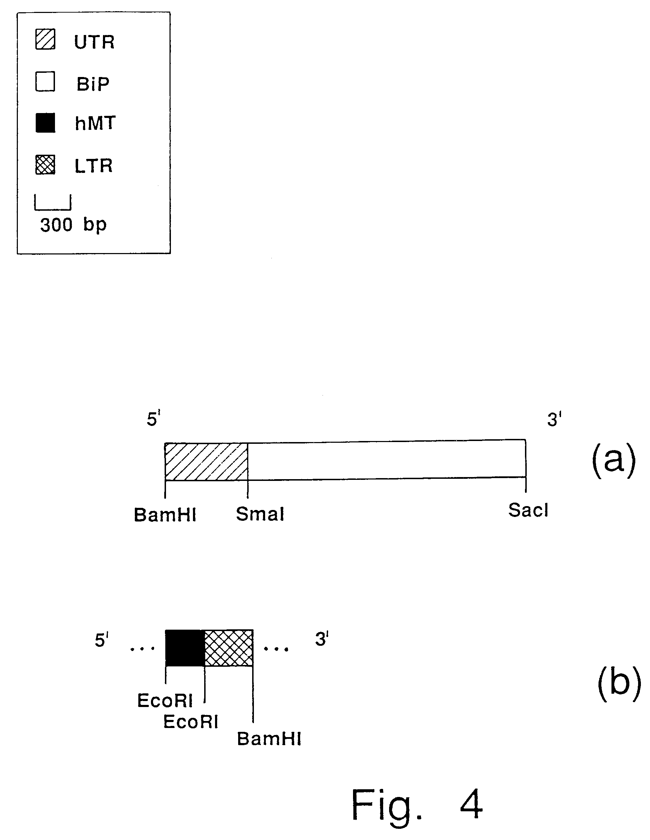

Two additional plasmids were constructed as described hereinafter (Fig. 4):

### Preparation of Construct 4(a)

A plasmid containing the UTR and BiP coding sequence was constructed. BamHI and SmaI sites were created at codons 1 and 698 of poliovirus cDNA, respectively. Additionally, SmaI and SacI sites were created at codons 57 and 2023 of BiP cDNA, respectively. The two fragments thereof, BamHI-SmaI containing the UTR, and SmaI-SacI containing BiP coding sequence, were ligated in frame into the cloning region of the pSP64 vector. The EcoRI site at the 3' end was modified into BamHI site as described above, and the composite BamHI-BamHI fragment containing UTR, BiP and poly A was inserted into the BamHI cloning of PM².

### Preparation of Construct 4(b)

To enforce an external control by adding metal ions to the condition medium, the enhancer domain from the metallothionein gene was inserted into PM² Construct 3(b), which contains the polio 5' UTR and the 2A protease. EcoRI sites were created at codons 1 and -300 of the metallothionein gene. The fragment EcoRI-EcoRI which contains the metallothionein control region was ligated into the EcoRI site of PM² at the first codon.

### B. Transfection and Clone Selection

The plasmids were transfected into the Chinese Hamster Ovary cell line (CHO) using the calcium-phosphate method as described previously [DeFeo, D. et al., Proc. Natl. Acad. si. U.S.A., 78, 3504(1981)]. The cells were maintained in medium I (Ham's F-12 medium supplemented with penicillin (100 units/ml), streptomycin (100 mg/µl) and glutamine (2 mM) containing 5% (v/v) of fetal calf serum at 37°C in a humidified 5% CO₂ incubator). Since the expression vector contains the neomycin resistance gene, transfected cells were selected by growing in culture medium containing 0.25 mg/ml of the neomycin analog G418.

The above constructs were employed for the following transfections:
1) Transfection of AcChoEase under the control of the LTR promoter (Construct 3(a)). The expression of AcChoEase in this transfection was through a cap-dependent mechanism and was used as a control for the other transfections.
2) Co-transfection of the capped AcChoEase (Construct 3(a)) together with the uncapped (cap-independent) UTR-2A (Construct 3(b)). This transformation leads to the inhibition of all cell proteins synthesis through cap-dependent mechanism, including AcChoEase.
3) Co-transfection of the uncapped UTR-AcChoEase (Construct 3(d)) together with the capped 2A (Construct 3(c)). The on-off translation of 2A protease by this transfection was through cap-dependent mechanism. However, translation of AcChoEase was through the uncapped mechanism.
4) Co-transfection of the UTR-AcChoEase (Construct 3(d)) and 5' UTR 2A (construct 3(b)). Expression and translation of 2A protease through cap-independent mechanism stopped protein synthesis of the host cells continuously, except for the translation of AcChoEase which was through cap-independent mechanism.
5) For uncapped expression together with BiP expression, transfection of UTR-BiP coding sequence (Construct 4(a)) was employed together with Constructs 3(d) and 3(b), all at one transfection.
6) In order to enforce an external control of expression, plasmids containing the enhancer domain of metallothionein gene (Construct 4(b)) were co-transfected together with Construct 3(d) (uncapped reporter). The transcription of the cDNAs was modulated by adding metals, such as zinc or copper, to the culture media.

### C. Metabolic Labelling

On day 0 transfected cells were placed into 12-well dishes (300,000 cells/well) in 1 ml of medium I (penicillin 100 µg/ml, streptomycin 100 µg/ml, glutamine 2mM, Ham's F12 medium) supplemented with 5% (v/v) fetal calf serum. On day 1, and for continuous labelling experiments, cells were washed twice with cysteine-free medium II (medium I supplemented with 5% dialyzed calf serum) and labelled for 7 hours in 1 ml of cysteine-free medium II containing 50 µCi/ml [³⁵S] cysteine (>1000 Ci/mmol, Amersham Corp.).

### D. Immunoprecipitation and Gel Electrophoresis

Medium was collected and chilled on ice. Stock buffer and detergent solutions were added so the final samples contained: 50 mM Tris-HCl (pH 7.8), 150 mM NaCl, 5 mM EDTA, 0.5% (vol/vol) Triton X-100, 0.1% NaDodSO₄, phenylmethyl-sulfonyl fluoride (0.3 mg/ml) and iodoacetamid (0.3 mg/ml).

Cell lysates were washed twice with phosphate buffer saline (4°C) and lysed by adding 0.2 ml of lysis buffer [50 mM Tris, pH 7.8,150 mM NaCl, 5 mM EDTA and 0.6% (wt/vol) NaDodSO₄]. The lysates were diluted with 0.8 ml of dilution buffer (same as lysis buffer, except 0.6% Triton X-100 was substituted for NaDodSO₄), and phenylmethyl-sulfonyl fluoride and iodoacetamide were added to a final concentration of 0.3 mg/ml.

The medium and lysate samples were precleared with normal rabbit serum (>1 hr.) and pansorbin (1 hr.) before the samples were immunoprecipitated. Polyclonal antisera were added to lysate and `media and incubated overnight at 4°C, as described [Laemmli, U. K., Nature 227, 680 (1970)]. The lysate and media immunoprecipitates were resolved on 15% SDS-polyacrylamide gels as described [Bonneau, A. M., and Sonenberg, N., J. Biol. Chem., 262, 11136 (1987)].

### E. Gel Electrophoresis and Peptide Maps

Autoradiography of the immunoprecipitation electrophoresis of the product showed that AcChoEase was produced under regular capped translation (transfection 1), and that uncapped internal translation shut-off conditions prevented the production of AcChoEase protein (transfection 2).

Quantitation by densitometry of AcChoEase proteins from transfections (3), (4) and (1) indicated that internal translations (3) and (4) produced 7 times as much AcChoEase proteins as the normal translation control (1). Transfection (5) (uncapped with BiP) gave results similar to transfections (3) and (4).

Immunoprecipitation electrophoresis of transfection 6 (external control) was performed and compared with transfection 2 (shut-off condition) and with transfection 1 (control).

Quantitation by densitometry of AcChoEase protein indicated that transfection 6 produced 8 times more protein than transfection 1 (control). However, the external control transfection 6 enabled to prolong the lifetime of the infected cell.

### F. Spectrophotometric Protein Detection

For studying the amount of expression and the biological activity of the reporter gene, AcChoEase medium was collected from the cell cultures. Before the collection of the media, cells were preincubated for 12 hours with medium which did not contain G418 and FCS, which might interfere with the bioassay. Medium was collected every 24 hours, clarified by centrifugation, and stored at -20°C until it was assayed for activity.

Expression of AcChoEase was spectrophotometrically detected by Ellman's method [G. L. Ellman et al., Biochem. Pharm., 7, 88 (1961)] for acetylthiocholine hydrolysis. 10 µl of sample were preincubated for 30-45 minutes in individual wells of a 96 well microtiter plate with Ellman's reagent (100 mM phosphate buffer, pH 7.0, 0.5 mM 5-5' dithiobis-2 nitrobenzoic acid, DTNB). The reaction was initiated by addition of 10 µl butyrylthiocholine (20% stock). Hydrolysis of substrate releases free acid and thiocholine. Thiocholine reacts at a molar ratio of 1:1 with DTNB to generate the yellow anion 5-thio-2-nitro-benzoic acid whose high extinction coefficient (E405=13600 M-1 - cm-1) renders this assay highly sensitive (path length approximately 0.5 cm). The kinetics of substrate hydrolysis was monitored by the Vmax automated microtiter plate reader (Molecular Devices Corp., USA) linked to IBM compatible computer equipped with specially adapted software for the calculation of rate constants. This assay accurately detects cholinesterase activities at nanomole levels of substrate.

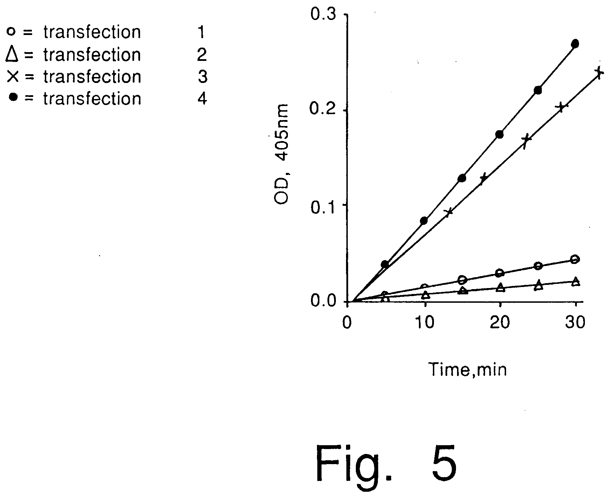

Figure 5 represents the average of three experiments and clearly indicates the increased production of AcChoEase protein in transfections 3 and 4 (internal translation mechanism coupled with shutoff translation of host cell proteins), compared to control transfection 1 (capped translation) and shut-off conditions - Transfection 2.

### Example 2

### Expression of Luciferase

### A. Construction of expression vectors

The same procedure as in Example 1 was employed, but instead of using the reporter gene Acetylcholinesterase, the reporter gene containing the cDNA of the firefly Luciferase was used. The cDNA of the Luciferase gene LC5'1811bp (J.R. Wet et al., Mol. Cell. Biol. (1987) 7, 725-737) was inserted into various constructs in PM² plasmid through BamHI as cloning site described in Example 1.

### B. DNA Transfections

Plasmid DNAs were transfected into CHO cells as described in Example 1. Plasmid DNA (10 µg) was used to transfect each 10 cm plate of cells, each plate containing approximately 10⁶ CHO cells. Cells were harvested 48 hours after transfection.

### C. Luciferase assays

Each plate of transfected cells was washed three times in phosphate buffer, and cells were harvested by extraction buffer (100 mM potassium phosphate (pH 7.8), 1 mM dithiothreidol). The cells (1-5 x10⁶) from each plate were pelleted and resuspended in 100 ml of extraction buffer.

Cells were lysed by three cycles of freezing on dry ice and thawing at 37°C. Cells debris were pelleted by centrifugation at 4°C. A 10 ml sample of extract was added to 350 ml of 25 mM of glycolglycine (pH 7.8) containing 5 mM ATP and 15 mM MgSO₄ in a small test tube. The tube was placed in an LKB luminometer equipped with recorder, and the reaction was initiated by the injection of 100µl of 1 mM luciferin. The enzyme catalyzed a rapid, ATP dependent oxidation of the substrate, which then emitted light at a rate proportional to the amount of luciferase present.

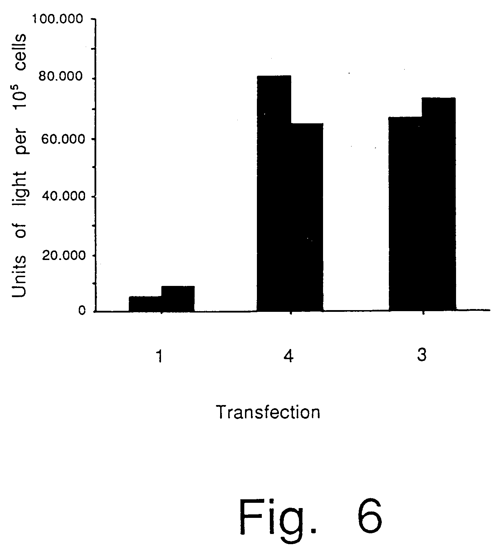

Luciferase activity obtained from extracts of CHO cells transfected with various constructs (as in Example 1) is shown in Fig. 6. Two separate experiments are shown. Transfection 3 and 4 produced 7 to 9 times more luciferase protein compared to control transfection 1.

### Example 3

### Expression of CAT

### A. Vector construction

Two stages of cloning are needed in order to obtain the desired constructs. In the first step the genes of choice (the 5'-UTR of the BiP gene and the poliovirus 2A protease) were amplified. The amplified fragments were cloned into the plasmid TA cloning vector of Invitrogen, which enables easy cloning of PCR amplified DNA. In the second stage of the cloning the amplified gene was transferred into the commercial pRc/CMV vector (of Invitrogen). This vector is based on the pUC 19 backbone and carries the strong CMV promoter, the T7 and Sp6 promoters for in vitro RNA synthesis, the SV40 polyadenylation signal, and the Neomycin and β-Lactamase genes for selection in bacteria (Amp) in mammalian (G418) cells. The cloning and recloning steps were performed by adding to the oligonucleotide primers restriction sites which are present in the polylinker of the vector but not in the genes of choice.

### 1) Cloning of the 5'-UTR of BiP (UTR)

The following primers were used for amplifying cellular DNA by PCR:
a) 5'-AACATCGCGGCCGCAGGTCGACGCCGGCCAAGACA-3' (Nuc. 372-392 of BiP + NotI site)
b) 5'-CATACGAAGCTTCTTGCCAGCCAGTTCGGCAGC-3' (Nuc. 592-572 of BiP + HindIII site).

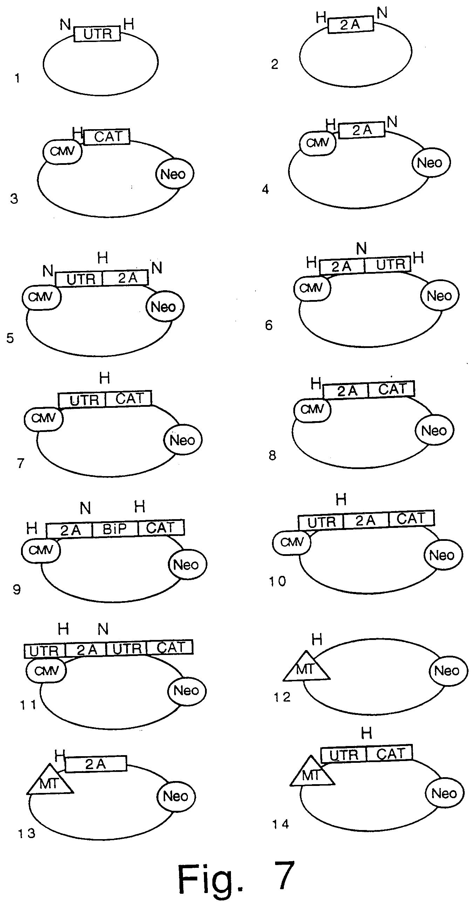

The 247 bp amplified band was cloned into the plasmid using the TA cloning kit of Invitrogen (plasmid 1 in Fig. 7).

### 2) Cloning of the poliovirus 2A protease

cDNA from the poliovirus RNA was prepared using the 18-mer primer 5'-ATTTGCTGGGTGAATCCA-3' (Nuc. 3890-3873 [La Monica et al., J. Virol. 57: 515-525 (1986)]).

The 2A protease gene was amplified by PCR from the cDNA using the primers:
a) 5'-TCCAACAAGCTTATGGGTTTTGGCCACCAAAAT (Nuc. 3382-3399, [La Monica et al., ibid] + ATG codon and HindIII site); and
b)5'CCTATCGCGGCCGCTTATTACTGCTCCATAGCCTCCTC-3' (Nuc. 3828-3811 [La Monica et al., ibid] + two stop codons and NotI site).

The 482 bp amplified band was cloned as before (plasmid 2 in Fig. 7).

### 3) Recloning of the CAT gene

The CAT gene was cut out from the pCAT basic plasmid of Promega (cat. #E1041) and was recloned into the HindIII site of the pRc/CMV plasmid of Invitrogen (plasmid 3 in Fig. 7). This plasmid carries the CMV promoter and the Neomycin (Neo) gene and was used as the reference plasmid for CAT activity in the transfected cells.

### 4) Cloning of combined constructs

Plasmids 1, 2 and 3 (Fig. 7) were used in the subsequent construction of plasmids 4 to 11 by means known to the skilled person. As an example, after propagating the plasmids in bacteria the BiP insert was isolated (from plasmid 1) using HindIII and NotI, and the CAT plasmid (plasmid 3) was linearized using HindIII. The HindIII site of the BiP insert was ligated (HindIII-HindIII) to the CAT gene; subsequently, the remaining free ends were blunt-ended and self-ligated. The result of these manipulations was plasmid 7 (UTR-CAT). HindIII ligation of the UTR fragment to 2A (plasmid 4) created plasmid 5, and NotI ligation of the same 2 DNAs gave plasmid 6. Since the 2A and the UTR genes are flanked by HindIII and NotI sites, construction of plasmids 8 to 11 took place in a way similar to that described above. Plasmids 3 to 11 therefore contained the following elements:
a) CMV-5'-CAT-3' (The CAT gene under CMV promoter; the above-mentioned plasmid 3) - Fig. 7.
b) CMV-5'-2A-3' (The 2A gene under CMV promoter; plasmid 4 in Fig. 7. This plasmid is the basic plasmid for the 2A protease expression).
c) CMV-5'-UTR-2A-3' (The 2A gene with the 5'-UTR under the CMV promoter; plasmid 5 in Fig. 7, a control for plasmid 4).
d) CMV-5'-2A-UTR-3' (Plasmid 6 in Fig. 7, needed as a control for the 2A activity and for the construction of dicistronic plasmids).
e) CMV-5'-UTR-CAT-3' (The CAT with 5'-UTR of the BiP; plasmid 7 in Fig. 7. This is the basic CAT plasmid for this experiment).

The following dicistronic plasmids were cloned, as well:
f) CMV-5'-2A-CAT (plasmid 8) - Fig. 7.
g) CMV-5'-2A-UTR-CAT-3' (plasmid 9) - Fig. 7.
h) CMV-5'-UTR-2A-CAT-3' (plasmid 10) - Fig. 7.
i) CMV-5'-UTR-2A-UTR-CAT-3' (plasmid 11) - Fig. 7.

The dicistronic plasmids provided positive and negative controls for transfection and expression efficiency under different conditions.

All the above-mentioned plasmids carry the CMV promoter, the Neomycin and β-lactamase genes (for selection in mammalian and bacterial cells), the reporter gene (CAT) with or without the 5'-UTR of the BiP, the poliovirus 2A protease, and combinations of the three DNA segments (genes).

### B. Cell transfections

Hela cells were transfected by electrophoration with each of the plasmids and with combinations of plasmids carrying the reporter gene (CAT) and the 2A gene.

### C. Assay of CAT Activity

After transfection, cell lysates were analyzed in order to estimate the quantities and stability of the mRNAs by Northern blots and hybridization. The quantities and efficiency of the CAT protein production were assayed as described in "C.N. Gorman et al., Mol. Cell Biol., 2, 1044 (1982)".

The cell extracts were incubated with radioactive chloroamphenicol and acetyl CoA. Acylated Chloroamphenicol was separated from the non-acetylated form by thin layer chromatography followed by autoradiography. CAT activity was determined as intensity at the origin position of unacetylated CAT and monoacetylated CAT products. The CAT activity resulting from the transfection of plasmids (7+5), (7 + 4), (9) and (11) varied between 500-800% of the activity detected in the transfection of plasmid (3) (control normal eukaryotic translation).

### Example 4

### Cloning of the MT-IIA Promoter

Example 3 was repeated, but the CMV promoter of the pRc/CMV vector was replaced by the inducible metallothionein (MT-IIA) promoter, to create a construct that can be induced to express the 2A protease under externally controlled conditions.

The MT-IIA promoter was amplified by using the following two primers:
a) 5'-ACCATTCGCGACACGGCGGAGGCGCACGGC-3' (Nuc. 1-20 of MT-IIA + NruI site); and
b) 5'-CCTAGGAAGCTTGATCATGGCGAGCTGAAGAGGC-3' (Nuc. 377-356 of MT-IIA + HindIII site).

This amplification yielded a 399 bp long fragment (377 bp of the MT-IIA promoter and two restriction sites) which were digested by the two above-mentioned enzyme to create a 384 bp long fragment which was cloned into the NruI/HindIII sites of the pRc/CMV plasmid.

The CMV promoter was removed from the pRc/CMV plasmid by digesting the plasmid with the NruI and HindIII restriction enzymes, thereby removing an insert of 686 bp (Nuc. 206 to 891 on the plasmid) that includes the CMV and the T7 promoters, but left intact the multicloning site. After cloning, the amplified MT-IIA promoter insert into the resulting linearized plasmid is the only promoter active in this construct (plasmid 12, Fig. 7).

The HindIII/NotI 2A protease segment from plasmid 2 (Fig. 7) was cloned into the HindIII of plasmid 12 thus crating plasmid 13 (Fig. 7) which carries the 2A gene under the control of the MT-IIA promoter. Plasmid 14 (Fig. 7) was cloned in two stages: first, the NotI/HindIII UTR insert from plasmid 1 was cloned into the HimdIII site of plasmid 12 (conserving the HindIII site but not the NotI site) and subsequently the CAT gene was cloned into the remaining HindIII site.

Cell transfections and tests were carried out as in Example 3 and comparable CAT activities were obtained when copper ions were added.

The above description and examples have been provided for the purpose of illustration and are not intended to limit the invention in any way. As will be understood by the skilled person, the invention encompasses all uses of the combined effect of the inhibition of cell protein production and of IRES-mediated expression of selected polypeptides, in order to obtain the desired effect, be it the industrial production of a desired material or the in vivo production of various materials for cell therapy in deficient cells, or for targeted cell destruction, or for any other purpose. The invention is not meant, therefore, to be limited to any specific cell, vector, IRES or protein, and a large variety of applications can be devised, without exceeding the scope of the invention or departing from its spirit.

## Claims

1. A method for the in vitro production of a gene product, comprising the steps of:
a) preparing one or more recombinant nucleotide vector(s) capable of eukaryotic cell transfection, collectively containing 1) a gene encoding a TIF of an eukaryotic translation factor (ETF), 2) at least one internal ribosome entry site (IRES) region, and 3) at least one gene encoding a polypeptide which it is desired to produce;
b) transfecting a eukaryotic cell culture with the said vector;
c) keeping the transfected cell culture under conditions suitable to promote cell activity; and
d) recovering the desired gene product from the said culture.

2. A method according to claim 1, wherein the ETF comprises the eIF-4F complex.

3. A method according to claim 1 or 2, wherein the recombinant nucleotide vector is selected from a DNA or an RNA vector.

4. A method according to claim 1, wherein the recombinant nucleotide vector comprises, in the same vector, 1) a sequence encoding a TIF preceded by a eukaryotic cap-dependent promoter; and 2) a sequence encoding the desired gene product preceded by an IRES.

5. A method according to claim 1, wherein the recombinant nucleotide vector comprises, in the same vector, 1) a sequence encoding a TIF preceded by an IRES; and 2) a sequence encoding the desired gene product preceded by an IRES.

6. A method according to any one of claims 1 to 5, wherein the gene encoding the TIF is the cDNA of the poliovirus 2A protease.

7. A method according to any one of claims 1 to 6, wherein the IRES region is that of a picornavirus or of BiP.

8. A method according to claim 7, wherein the picornavirus is the polio virus or EMCV.

9. A method for the in vitro production of a gene product, comprising the steps of:
a) preparing a first recombinant nucleotide vector, comprising a sequence encoding a TIF, preceded by a eukaryotic cap-dependent promoter;
b) preparing a second recombinant nucleotide vector, comprising at least a sequence encoding the desired gene product, preceded by an IRES;
c) transfecting a eukaryotic cell culture with both the said first vector and the said second vector;
d) keeping the transfected cell culture under conditions suitable to promote cell activity; and
e) recovering the desired gene product from the said culture.

10. A method for the in vitro production of a gene product, comprising the steps of:
a) preparing a first recombinant nucleotide vector, comprising a sequence encoding a TIF, preceded by an IRES;
b) preparing a second recombinant nucleotide vector, comprising at least a sequence encoding the desired gene product, preceded by an IRES;
c) transfecting a eukaryotic cell culture with both the said first vector and the said second vector;
d) keeping the transfected cell culture under conditions suitable to promote cell activity; and
e) recovering the desired gene product from the said culture.

11. A method according to any one of claims 1 to 10, further comprising providing for external activation of the TIF expression.

12. A method according to claim 11, wherein the external activation of TIF expression is mediated by metallothionein activity.

13. A method according to any one of claims 4 to 12, wherein the recombinant nucleotide vector is selected from a DNA or an RNA vector.

14. A method according to any claim 13, wherein the recombinant nucleotide vector is a recombinant DNA vector, comprising the DNA sequence of 2A protease.

15. A method according to any one of claims 1 to 14, wherein the gene product is a polypeptide.

16. A method according to any one of claims 1 to 14, wherein the gene product is a protein.

17. A recombinant nucleotide vector comprising a gene encoding a TIF, preceded by a eukaryotic cap-dependent promoter.

18. A recombinant nucleotide vector comprising a gene encoding a TIF, preceded by an IRES.

19. A recombinant nucleotide vector comprising a sequence encoding a useful gene product, preceded by an IRES.

20. A recombinant nucleotide vector comprising in sequence, as part of the genetic material, a gene encoding a TIF, an IRES and at least one gene encoding a useful gene product.

21. A vector according to claim 20, wherein the TIF is preceded by a eukaryotic cap-dependent promoter.

22. A vector according to claim 20, wherein the TIF is preceded by an IRES.

23. A vector according to any one of claims 17 to 22, wherein the gene encoding the TIF is the cDNA of the poliovirus 2A protease.

24. A vector according to any one of claims 17 to 23, wherein the IRES region is that of a picornavirus or of BiP.

25. A vector according to claim 24, wherein the picornavirus is the polio virus or EMCV.

26. A vector according to any one of claims 17 to 25, further comprising a metallothionein promoter.

27. A vector according to any one of claims 17 to 26, which is a DNA or an RNA vector.

28. A pharmaceutical preparation comprising a vector according to any one of claims 17 to 27.

29. A method of effecting in vivo gene therapy, comprising treating a patient in need thereof with a pharmaceutical preparation according to claim 28.

30. A method for the extracellular production of a gene product, comprising the steps of:
a) preparing a medium containing a recombinant nucleotide vector comprising at least one sequence encoding the desired gene product, preceded by an IRES;
b) providing in the medium substrates, ribosomes and promotors required for effecting translation;
c) keeping the medium under conditions suitable to promote gene expression; and
d) recovering the desired gene product from the said medium.

31. A method according to claim 30, further comprising preparing a recombinant nucleotide vector, which may be the same or separate from vector (b) of claim 30, comprising a sequence encoding a TIF, preceded by an IRES, and adding such vector to the said medium.

32. A gene product, whenever prepared by the method of any one of claims 1 to 16 or 30-31.
